# EUROPEAN PATENT APPLICATION

(11) **EP 2 452 718 A1**
(43) Date of publication of application: **16.05.2012**
(21) Application number: 10797132.7
(22) Date of filing: 06.07.2010
(51) Int. Cl.: A61M 25/00, A61F 9/007, A61M 25/01

(54) **INSERTION AID FOR MEDICAL DEVICES AND MEDICAL DEVICE USING SAID INSERTION AID**

(30) Priority: 06.07.2009 JP 2009160276
(71) Applicant: Kaneka Corporation, Osaka-shi, Osaka 530-8288 (JP)
(72) Inventor: FUKAYA, Kohei, Settsu-shi Osaka 566-0072 (JP)
(74) Representative: TBK
(86) International application number: PCT/JP2010/061476
(87) International publication number: WO 2011/004821

(57) **Abstract**

Medical devices that are inserted into a body and that are used for treating a lumen inside a body sometimes use an insertion aid during insertion. However, there was a tendency for the relative positions of the insertion aid and the medical device to change, causing problems such as reduced operability and the insertion aid breaking the medical device during insertion. Disclosed are an insertion aid used by being placed within a medical device, and a medical device using said insertion aid. The insertion aid has a mechanism for holding said medical device from the inside, and said medical device uses said insertion aid, thereby making it possible to fix the relative positions of the insertion aid and the medical device when necessary.

## Description

### Technical Field

The present invention relates to an insertion aid which is placed and used in a medical device which is inserted into a body and used for treating a lumen in the body, and medical device using the insertion aid.

### Background Art

An angioplasty for improving blood flow in a peripheral blood vessel by dilating the stenosis lesion or obstruction lesion in the blood vessel when stenosis or obstruction occurs in a vascular vessel such as a blood vessel (PTA: Percutaneous Transluminal Angioplasty; PTCA: Percutaneous Transluminal Coronary Angioplasty; and the like) has become general as an operation in this kind of cases. A dilatation catheter in PTA and PTCA is used together with a guide catheter and a guide wire as a set, in order to mainly dilate the stenosis lesion or the obstruction lesion in the blood vessel. The angioplasty using the dilatation catheter is generally performed as follows: First, a guide catheter is inserted from a femoral artery and passes through an aorta, and its tip is placed at an entrance of a coronary artery. Next, only a guide wire, which has been previously inserted into a dilatation catheter, is caused to move forward over a stenosis lesion or a obstruction lesion in the blood vessel, and then a dilatation catheter is caused to move forward along the guide wire. After that, a dilatation part (balloon) is expanded in a state in which it is placed at the stenosis lesion or the obstruction lesion to dilate the stenosis lesion or the obstruction lesion. After the dilatation of the stenosis lesion or the obstruction lesion, the dilatation part is shrunk and removed from the body.

The use of this dilatation catheter is not limited to the treatment of the stenosis lesion or the obstruction lesion in the blood vessel, and is useful in various medical applications including insertion into a blood vessel as well as insertion into various body cavities and tubular tissues. However, in a case in which it is used for treating a blood vessel, for example, when the degree of the stenosis progresses and it is difficult to pass through the stenosis lesion for the guide wire, a catheter for treating a stenotic vessel such as a straight microcatheter or a tapered microcatheter or dilatation catheter is sometimes used together with the guide wire. Even if they are used together with the guide wire, however, neither the obtained effects nor ease of use is sufficient. In addition, even if the guide wire passes through the stenosis lesion, it is sometimes difficult to cause the dilatation catheter to move forward along the guide wire. An effective mechanism or aid in such a case has been desired.

On the other hand, with respect to a treatment for lumens other than the blood vessel, a treatment method in which an obstruction site in a lacrimal duct from a lacrimal canaliculus to an inferior nasal meatus is dilated by using a dilatation catheter for treating a lacrimal duct having a dilatation part which is expanded with pressure; and a treatment method in which a bougie is inserted into a lacrimal duct to remove an obstruction site, then a lacrimal duct tube is inserted into the lacrimal duct by using a stylet-shaped insertion aid, next the insertion aid is removed to indwell the lacrimal duct tube in the lacrimal duct for a predetermined time to prevent the re-obstruction, are adopted. In these treatments, it is necessary to insert the dilatation catheter for treating a lacrimal duct or the lacrimal duct tube into the curved lacrimal duct. These tubes are required to have contrary properties, that is, a property capable of applying force for insertion (that is, rigidity of a tube) and tip-softness for preventing breaking through a wall surface of the lacrimal duct. It is not always easy, however, to provide the contrary properties to the tube itself, and therefore, an insertion aid may be sometimes used. For example, a lacrimal duct tube may be used in a state in which the tip thereof is softened by inserting halfway an insertion aid into the lacrimal duct tube for softening the tip upon the insertion into the lacrimal duct. In such a case, the relative position to the lacrimal duct tube easily shifts, and the operability is problematically reduced. Although it is often preferable to keep the tip of the lacrimal duct tube open, this sometimes cannot be performed, because the insertion aid tends to penetrate through the tip end area of the lacrimal duct tube upon the insertion. In addition, because of the tendency of the easy penetration upoon the insertion, as described above, damages in which the aid breaks through the tip end of the lacrimal duct tube or another medical device may occur.

Although its object and structure are different from those of the present invention, Patent Document 1 discloses an anchor-type guide wire having an ejection part which serves to fix a guide wire to an organ wall of a living body. This technique is **characterized in that** a sheath made of a metal tube previously houses a core wire having an original shape, which projects outside from the sheath in a free space, and the core wire is slid from the sheath and exposes at a desired position, and the core wire is slide back and housed again in the sheath upon the recovery. In this case, very great force is necessary to take the wire into and out from the sheath having a diameter smaller than that of the original shape of the core wire by sliding the wire. On the contrary, when a slidable core wire is designed within a practical range, the original shape which has projected outside becomes very weak. According to the technique described in Patent Document 1, accordingly, though the anchor effect to an organ wall of a living body may be observed sometimes, the ability to hold a medical device from the inside thereof when the medical device is inserted into the body, which is the purpose of the present invention, is quite insufficient.

### Citation List

### Patent Literature

Patent Document 1: JP-A No.8-71158

### Summary of Invention

### Technical Problem

The present invention provides an insertion aid which is placed and used in a medical device which is inserted into a body and used for treating a lumen in the body, wherein the aid is capable of holding the medical device from the inside with sufficient force, when it is desired to fix the relative positions of the insertion aid and the medical device, and a medical device using the same.

### Solution to Problem

In order to solve the problems described above, painstaking studies have been made. As a result, it has been found that in a case in which an insertion aid is placed inside a medical device, when the insertion aid has a function of holding the medical device from the inside, the problems can be solved, and the present invention has been completed.

That is, the gists of the present invention are as follows:
(1) An insertion aid which is placed in a medical device and is used at the time when the medical device is inserted into a body, comprising a mechanism for holding the medical device from the inside.

(2) The insertion aid according to (1) described above, wherein the holding mechanism is a mechanism in which a part of the insertion aid having a predetermined diameter protrudes in relation to the predetermined diameter.

(3) The insertion aid according to (2) described above, wherein the holding mechanism is a mechanism in which a coiled spring having a predetermined diameter is placed on a part of the insertion aid, and the spring is axially compressed whereby it protrudes in relation to the predetermined diameter.

(4) The insertion aid according to (2) described above, wherein the holding mechanism is a mechanism in which a rubber elastic tubular member having a predetermined diameter is placed on a part of the insertion aid, and the tubular member is axially compressed whereby it protrudes in relation to the predetermined diameter.

(5) The insertion aid according to (2) described above, wherein the holding mechanism is a mechanism in which a spirally cut tube having a predetermined diameter is placed on a part of the insertion aid, and the spirally cut tube is radially unwound whereby it protrudes in relation to the predetermined diameter.

(6) The insertion aid according to (2) described above, wherein the holding mechanism is a mechanism in which a balloon expandable by pressure is placed on a part of the insertion aid, and a pressure is introduced into the balloon whereby it protrudes in relation to the predetermined diameter.

(7) The insertion aid according to any one of (1) to (6) described above, wherein the holding mechanism is a mechanism in which an area having a predetermined diameter placed on a part of the insertion aid is deformed by operation of a control unit placed on a base end whereby it protrudes in relation to the predetermined diameter.

(8) A medical device comprising the insertion aid according to any one of (1) to (7) described above.

(9) The medical device according to (8) described above, wherein the medical device is a catheter for treating a stenotic vessel.

(10) The medical device according to (8) described above, wherein the medical device is a dilatation catheter for treating a lacrimal duct.

(11) The medical device according to (8) described above, wherein the medical device is a tube for treating a lacrimal duct.

### Advantageous Effects of Invention

The insertion aid which is placed and used in a medical device when the medical device is inserted into a body, and which has a mechanism for holding the medical device from the inside has been invented. When it is desired to fix the relative positions of the insertion aid and the medical device, the insertion aid according to the present invention can hold the medical device with sufficient strength. Consequently, for example, catheters for treating a stenotic vessel having further improved response capability to a highly stenotic part of a vessel, and dilatation catheters for treating a lacrimal duct and tubes for treating a lacrimal duct having further improved operability and safety can be obtained.

### Brief Description of Drawings

Fig. 1 is a schematic view showing one embodiment of a guide wire, which is an insertion aid according to the present invention used in a catheter for treating a stenotic vessel.
Fig. 2 is a schematic view showing one embodiment in which a guide wire, which is an insertion aid according to the present invention used in a catheter for treating a stenotic vessel, is in a state in which the guide wire holds the medical device from the inside.
Fig. 3 is a schematic view illustrating an embodiment of an insertion aid and a medical device containing the same, according to the present invention, which device is a catheter for treating a stenotic vessel (dilatation catheter).
Fig. 4 is a schematic view showing another embodiment of a guide wire, which is an insertion aid according to the present invention used in a catheter for treating a stenotic vessel.
Fig. 5 is a schematic view showing one embodiment in which a guide wire, which is an insertion aid according to the present invention, is in a state in which the guide wire holds a medical device from the inside.
Fig. 6 is a schematic view illustrating an embodiment of an insertion aid and a medical device containing the same, according to the present invention, which device is a catheter for treating a stenotic vessel (microcatheter).
Fig. 7 is a schematic view showing an insertion aid (bougie) according to the present invention used in a medical device for treating a lacrimal duct.
Fig. 8 is a schematic view showing an insertion aid (bougie) according to the present invention used in a medical device for treating a lacrimal duct.
Fig. 9 is a schematic view showing an insertion aid (bougie) used in a medical device for treating a lacrimal duct, according to the present invention.
Fig. 10 is a schematic view showing an insertion aid (bougie) according to the present invention used in a medical device for treating a lacrimal duct.
Fig. 11 is a schematic view showing an insertion aid (bougie) according to the present invention used in a medical device for treating a lacrimal duct.
Fig. 12 is a schematic view showing an insertion aid (bougie) according to the present invention used in a medical device for treating a lacrimal duct.
Fig. 13 is a schematic view showing an insertion aid (bougie) according to the present invention used in a medical device for treating a lacrimal duct.
Fig. 14 is a schematic view showing an insertion aid (bougie) according to the present invention used in a medical device for treating a lacrimal duct.
Fig. 15 is a schematic view illustrating a state in which an insertion aid (bougie) according to the present invention used in a medical device for treating a lacrimal duct is placed inside of a dilatation catheter for treating a lacrimal duct, and holds the dilatation catheter for treating a lacrimal duct from the inside.
Fig. 16 is a schematic view of a tip end showing a state in which an insertion aid (bougie) according to the present invention used in a medical device treating a lacrimal duct is placed inside of a tube for treating a lacrimal duct, and holds the tube for treating a lacrimal duct from the inside.

### Description of Embodiments

Embodiments of insertion aids and medical device containing the same according to the present invention will be explained below.
In the explanations below, a guide wire and a bougie are shown as typical examples of the insertion aid, but the constitution of the present invention can be applied to insertion aids other than the guide wire and bougie within a range where the effects of the present invention can be obtained. In addition, the medical device will be explained with several concrete examples, but the insertion aid of the present invention can be applied to various medical devices within a range where the effects of the present invention can be obtained, as the same as above.

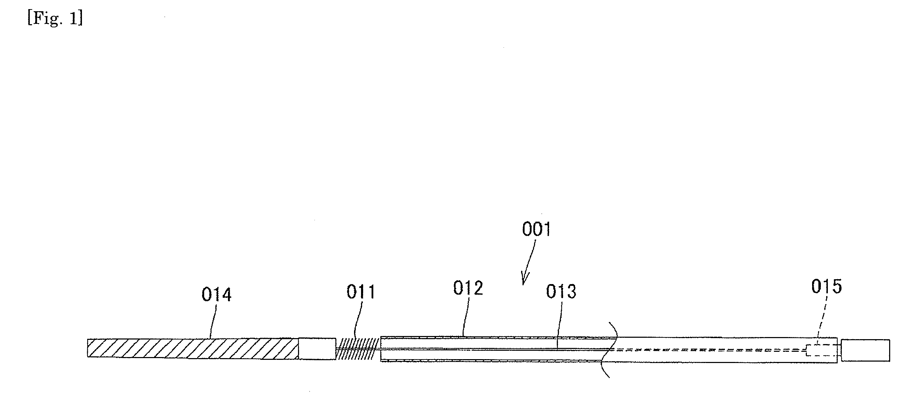

Figs. 1 to 3 show a first embodiment of an insertion aid of the present invention.
Fig. 1 is a whole schematic view showing guide wire 001 which is an insertion aid according to the present invention used in a catheter for treating a stenotic vessel. Coiled spring 011 having a predetermined diameter is placed between body 012 of the guide wire which is an insertion aid used in a catheter for treating a stenotic vessel and flexible tip area 014 thereof. In addition, linear member 013 is connected to flexible tip area 014 and control unit 015 placed on a base end, and is placed so that it passes through inside of body 012 and the inside of coiled spring 011. Coiled spring 011 having a predetermined diameter is placed between body 012 of the guide wire and flexible tip area 014 thereof so that it has the predetermined diameter (size) which is the same as their outer diameters.

The position of coiled spring 011 may be appropriately decided depending on the medical device used and the using mode so that it is placed inside the medical device. The position is not limited to the tip end of insertion aid 001 as shown in Figs. 1 to 3, and it may be placed at any place such as an intermediate area or a base end area. When coiled spring 011 is placed at the intermediate area or the base end area, body 012 may be divided into two areas, and a flexible tip area may be placed at the most tip end of the tip side. Linear member 013 may be connected to the flexible tip area or to the base end area of the tip side of body 012.
The flexible tip area serves to improve safety by providing flexibility to the tip end area of guide wire 001. For this reason, the shape, structure, size, and material thereof are not particularly limited so long as the flexibility can be secured, and known structures such as single lines, coiled wires, and braid lines made of a metal or resin may be adopted. This point applies to other embodiments (guide wires and bougies) described below.

Here, it is preferable to decide the predetermined diameter based on an area where the vertical length to the longitudinal direction of the medical device is the maximum in an area where insertion aid 001 is placed inside. The reason therefor is that the area where the vertical length is the maximum is a standard to determine whether the insertion into the inside of the medical device can be performed or not. If the insertion into the inside of the medical device can be secured, the predetermined diameter may be larger than the area where the vertical length is the maximum. It is desirable, however, that the predetermined diameter is equal to that of the area where the vertical length is the maximum, in terms of the operability of the medical device and the insertion aid when they are inserted into a body. These points apply to other embodiments below.

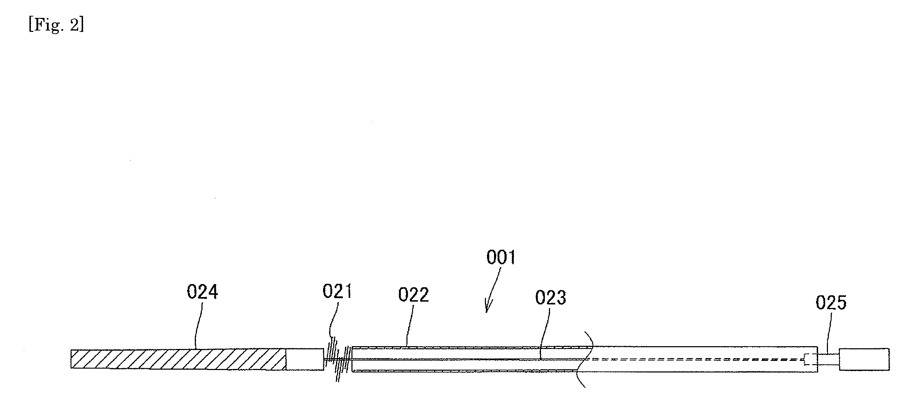

Fig. 2 is a whole schematic view of guide wire 001 which is the insertion aid according to the present invention used in a catheter for treating a stenotic vessel, and shows a state in which coiled spring 021 having the predetermined diameter protrudes in relation to the predetermined diameter by compression in the axial direction of guide wire 001.

Here, the phrase "protruded in relation to the predetermined diameter" means that the diameter of area (011 (021)) having the predetermined diameter in insertion aid 001 is larger than the predetermined diameter. When the diameter becomes larger as above, area (021 (011)) is brought into pressure contact with a wall forming the inside of a catheter for treating a stenotic vessel, which is the medical device into which guide wire 001 is inserted. As a result, guide wire 001, which is the insertion aid, is fixed within the catheter for treating a stenotic vessel with a sufficient force. This mechanism enables guide wire 001 to hold the catheter for treating a stenotic vessel from the inside. These points apply to other embodiments below.

As shown in Fig. 2, in this embodiment, control unit 025 moves relative to body 022 toward the base end, while it pulls linear member 023 and flexible tip area 024 connected thereto. A structure to transform control unit 025 relative to body 022 and a structure to maintain the pull, which is formed if necessary, are not particularly limited. The structure to maintain the pull has preferably a mechanism in which the pull is easily released so that the guide wire can be removed from the catheter when the guide wire becomes unnecessary.

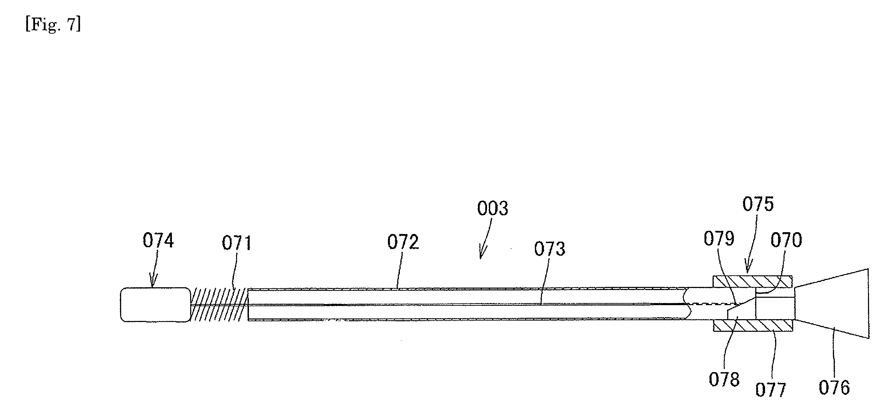

Known mechanisms may be appropriately applied to such a mechanism to maintain or release of the pull. Examples thereof include a mechanism in which control unit 025 and body 022 are screwed; a mechanism in which a depression and a protrusion, which engage each other, are made on control unit 025 and body 022 respectively, and the engagement and the release of the protrusion and the depression is performed by rotating control unit 025 to body 022; a mechanism in which, as the same as above, a depression and a protrusion are made, and control unit 025 is slid in an axial direction of body 022, whereby the protrusion moves in an almost vertical direction to the axial direction to perform the engagement of the protrusion with the depression or release the engagement; a mechanism in which a small hole which passes completely through from body 022 to control unit 025 is made in an almost vertical direction to the longitudinal direction of body 022 when control unit 025 is moved by pulling it toward the base end, and a pin is inserted into or pulled out from the hole; and a mechanism in which when a gap is formed between the terminal of the base end area of body 022 and the control unit, a member which fits the gap is inserted into or pulled out from the gap, and the like. In addition, a structure of a control unit shown in Fig. 7, or the like, described below, may be employed.

In the first embodiment, coiled spring 021 having the predetermined diameter is axially compressed by narrowing the space between body 022 and flexible tip area 024, and each element of the winding wire of coiled spring 021 shifts (that is, deforms) to linear member 023, whereby the coiled spring protrudes in relation to the predetermined diameter (that is, area (011 (021)) having the predetermined diameter expands in a vertical direction to the axial direction of the guide wire). Properties of coiled spring 021 such as a material, a coil diameter, a wire diameter, wire turns, and a spring constant should be appropriately decided depending on the insertion aid used and the medical device containing the same. If the spring constant is insufficiently large, however, the holding force cannot be obtained. It is, thus, at least 1 gf/mm (9.8 × 10⁻³ N/mm), preferably 5 gf/mm (49 × 10⁻³ N/mm) or more. If there is an insufficient clearance between the outer diameter of linear member 023 and the inner diameter of coiled spring 021, the elements of the winding wire do not shift when the spring is compressed and thus the protrusion insufficiently occurs. For this reason, the difference between the outer diameter of linear member 023 and the inner diameter of coiled spring 021 is preferably equal to or more than an outer diameter of the coil forming coiled spring 021. In order to sufficiently protrude the spring, the compression amount on the operation is preferably 1/3 the length of coiled spring 021 placed or more.

Coiled spring 011 (021), body 012 (022), linear member 013 (023), flexible tip area 014 (024), and control unit 015 (025), which are the members forming the guide wire in this embodiment, can be appropriately produced using known materials such as metals and various resins.

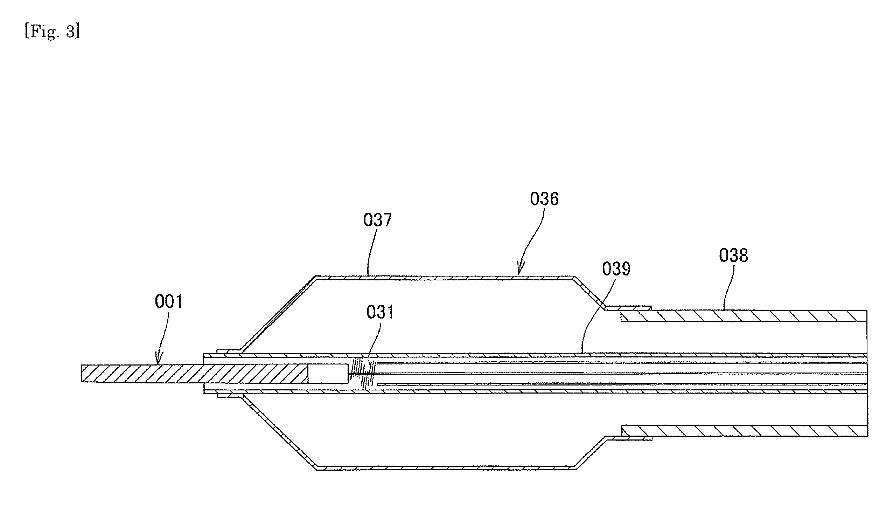

Fig. 3 is a schematic view of a tip end part showing a state in which guide wire 001, which is the insertion aid according to the present invention used in the catheter for treating a stenotic vessel, is placed inside catheter 036 for treating a stenotic vessel, and holds dilatation catheter (036), which is the catheter for treating a stenotic vessel, from the inside. Dilatation catheter (036) is composed of dilatation part 037 for dilating a stenotic area, outer tube 038, and inner tube 039 for passing (inserting) guide wire 001. The state in which coiled spring 031 in guide wire 001 placed inside (in this embodiment, in an inner space of inner tube 039) protrudes in relation to the predetermined diameter, and presses inner tube 039 (that is, it is brought into pressure contact with the wall surface of inner tube 039), whereby the spring holds the catheter from the inside, is shown. When the structure is formed described above, it is possible to easily keep a relative position between guide wire 001 which is the insertion aid and catheter 036 for treating a stenotic vessel which is the medical device with a sufficient strength.

In this embodiment, when the state in which each element of the winding wire of coiled spring 031 shifts is released by using the control unit (it is not shown in Fig. 3), the state can be easily returned to the original state of the coiled spring having the predetermined diameter (the state shown in Fig. 1). When guide wire 001 becomes unnecessary, therefore, it can be also easily removed therefrom. As described above, by operating the control unit, area (011 (031)) having the predetermined diameter can be deformed, whereby it protrudes in relation to the predetermined diameter (that is, the area (011 (031)) having the predetermined diameter expands in a vertical direction to the axial direction of guide wire 001), or on the contrary, the protrusion can be released, that is, the area can return from the state in which the area is expands in the vertical direction to the original size.

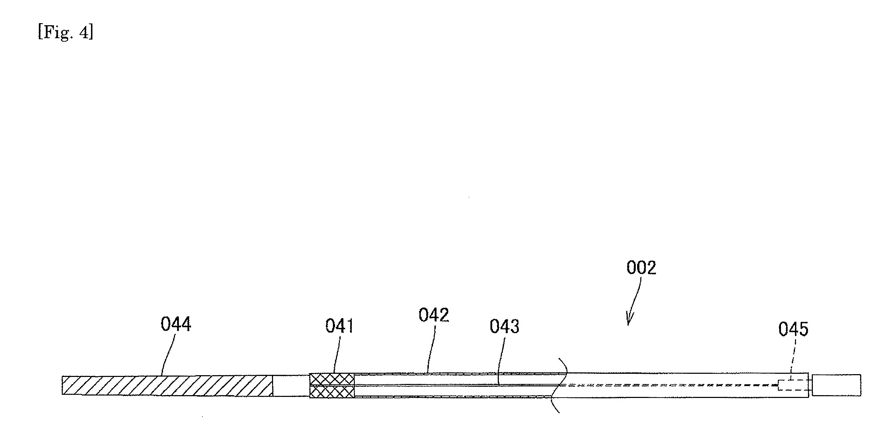

Figs. 4 to 6 show a second embodiment of an insertion aid according to the present invention.
Fig. 4 is a whole schematic view showing guide wire 002 which is an insertion aid according to the present invention used in a catheter for treating a stenotic vessel. Rubber elastic tubular member 041 having a predetermined diameter is placed between body 042 of guide wire 002, which is the insertion aid used in a catheter for treating a stenotic vessel, and flexible tip area 044 of the body, through which linear member 043 which is connected to flexible tip area 044 and control unit 045 placed on the base end and passes through the inside of body 042, passes. Tubular member 041 having the predetermined diameter is placed between body 042 of guide wire 002 and flexible tip area 044 so that it has the predetermined diameter, which is the same as their outer diameters (sizes).

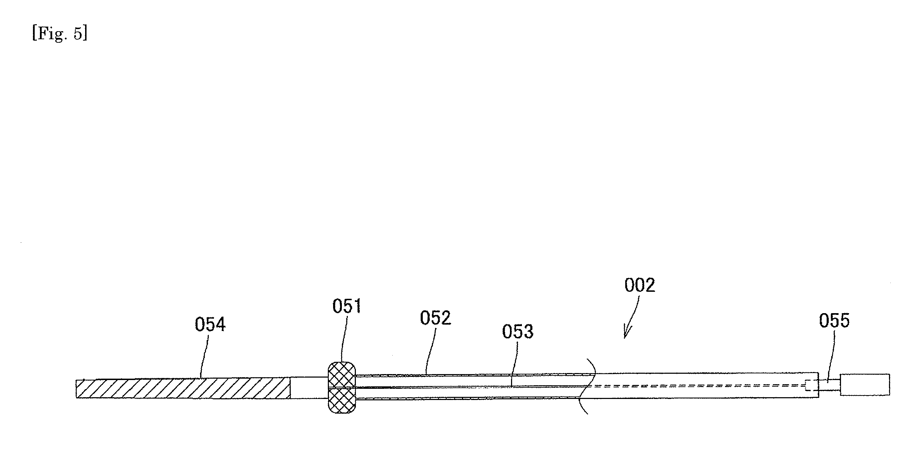

Fig. 5 is a whole schematic view of guide wire 002 which is the insertion aid according to the present invention used in a catheter for treating a stenotic vessel, and shows a state in which tubular member 051 having the predetermined diameter is compressed in an axial direction of guide wire 002, whereby it protrudes in relation to the predetermined diameter.

As shown in Fig. 5, control unit 055 moves relative to body 052 toward the base end, while it pulls linear member 053 and flexible tip area 054 connected thereto. The same structures as in the first embodiment are also applicable to the structure to transfer control unit 055 relative to body 052 in the second embodiment.

In the second embodiment, tubular member 051 having the predetermined diameter is axially compressed by narrowing a space between body 052 and flexible tip area 054, and tubular member 051 deforms so that it expands in the diameter direction, whereby the tubular member protrudes in relation to the predetermined diameter (that is, area (041 (051)) having the predetermined diameter expands in a vertical direction to the axial direction of the guide wire). Properties of tubular member 051 such as a material, inner and outer diameters, and a length should be appropriately decided depending on the insertion aid used and the medical device containing the same. As it is desired to return the diameter to the original predetermined diameter again after the deformation, rubber elastic materials are preferable. If the elastic modulus of the rubber elastic material is not small to some extent, sufficient protrusion cannot occur with respect to the compression. On the contrary, it is too small, holding force cannot be obtained. In view of these points, the bending elastic modulus is preferably 285 MPa or less and 5 MPa or more, and it is more preferably 170 MPa or less and 12 MPa or more. The surface softness of the rubber elastic material may be appropriately selected based on the properties of the inside wall surface of the medical device to be held, and when the material has softness to some extent, friction easily occurs and the holding property may be sometimes improved. The Shore D hardness, therefore, is preferably 64 or less, more preferably 27 or less. The bending elastic modulus and the Shore D hardness can be determined in accordance with JIS K 7171 and JIS K 6253, respectively.

Body 042 (052), linear member 043 (053), flexible tip area 044 (054), and control unit 045(055) which are the members forming guide wire 002 in the second embodiment, can be appropriately produced using known materials such as metals and various resins, similar to the first embodiment.

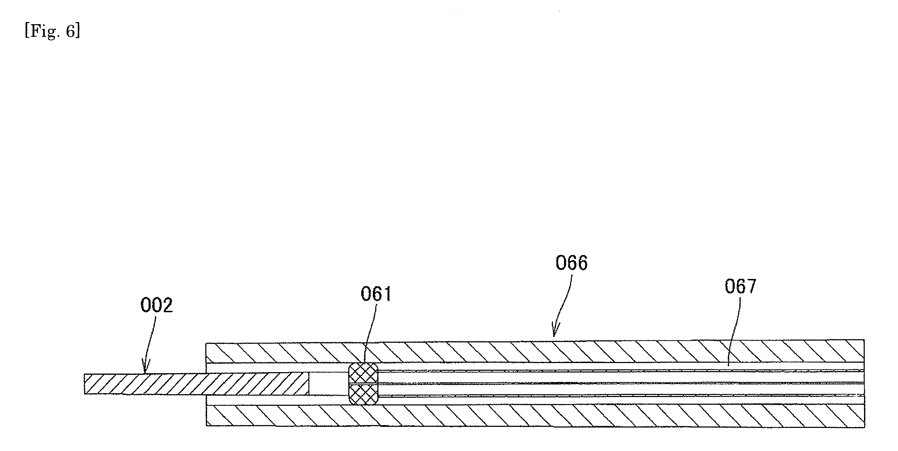

Fig. 6 is a schematic view of a tip end part showing a state in which guide wire 002, which is the insertion aid according to the present invention used in the catheter for treating a stenotic vessel, is placed inside of catheter 066 for treating a stenotic vessel, and holds microcatheter (066), which is the catheter for treating a stenotic vessel, from the inside. Passage 067 for passing (inserting) guide wire 002 is provided in microcatheter (066). The state in which tubular member 061 of guide wire 002 placed in passage 067 protrudes in relation to the predetermined diameter (that is, area (041 (061) having the predetermined diameter) expands in a vertical direction to the axial direction of the guide wire), and presses passage 067 for passing guide wire 002 (that is, it is brought into pressure contact with the inside wall surface forming passage 067), whereby the tubular member holds microcatheter (066) from the inside, is shown. When the structure is formed described above, it is possible to fix a relative position between guide wire 002, which is the insertion aid, and catheter 066 for treating a stenotic vessel, which is the medical device, and to easily hold catheter 066 with sufficient strength.

In this embodiment, the tubular member which deforms so that it expands in the diameter direction can be easily returned to the original state of the tubular member having the predetermined diameter (the state shown in Fig. 4), by the control unit (it is not shown in Fig. 6). When guide wire 002 becomes unnecessary, therefore, it can be easily removed therefrom. As described above, by operating the control unit, area (041 (061)) having the predetermined diameter can be deformed, whereby it protrudes in relation to the area (that is, area (041 (061)) having the predetermined diameter expands in a vertical direction to the axial direction of guide wire 002), or on the contrary, the protrusion can be released, that is, the area can return from the state in which the area is expands in the vertical direction to the axial direction of guide wire 002 to the original size.

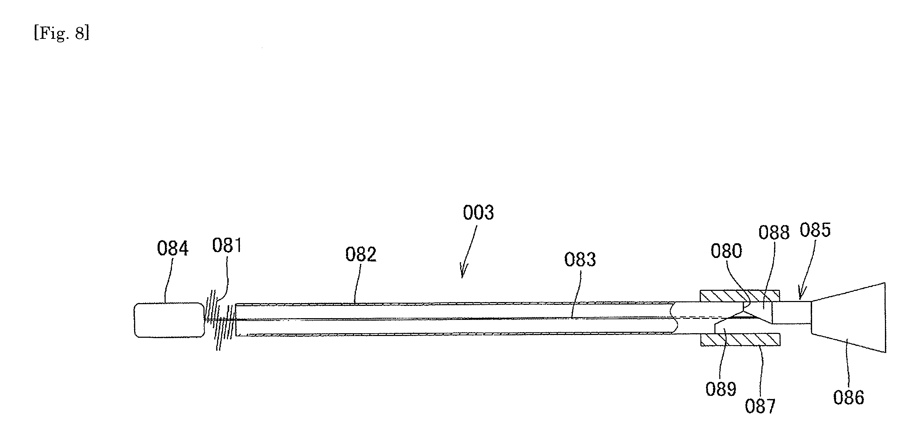

Figs. 7 and 8 show a third embodiment of an insertion aid according to the present invention.
Fig. 7 is a whole schematic view of bougie 003 which is an insertion aid according to the present invention used in a medical device treating a lacrimal duct. Coiled spring 071 having a predetermined diameter is placed between body 072 of insertion aid (bougie) 003 used in a medical device treating a lacrimal duct and tip end area 074, through which linear member 073 which is connected to tip end area 074 and control unit 075 placed on the base end and passes through the inside of body 072, passes. Coiled spring 071 having the predetermined diameter is placed between body 072 and tip area 074 thereof so that it has the predetermined diameter, which is the same as their outer diameters (sizes).

Control unit 075 is composed of shank 076 and outer tube portion 077. Shank 076 has, on its tip side, engaging portion 078 having a shape engaging with notch 079 which is provided in the base end side of body 072. The base end area of body 072 is inserted into outer tube portion 077, and both are fixed. On the other hand, shank 076 is movable in parallel to and relative to an axial direction of outer tube portion 077, and is rotatably engaged with outer tube portion 077 around a central axis in the axial direction of outer tube portion 077. Linear member 073 is joined to shank 076 on the base end side.

Fig. 8 is a whole schematic view of insertion aid (bougie) 003 according to the present invention used in a medical device treating a lacrimal duct, and shows a state in which coiled spring 081 having a predetermined diameter is compressed in an axial direction of insertion aid (bougie) 003, whereby it protrudes in relation to the predetermined diameter.

In Fig. 8, control unit 085 (in particular, shank 086) moves relative to body 082 toward the base end, while it pulls linear member 083 and tip end area 084 connected thereto. In this embodiment, the structure in which control unit 085 (in particular, shank 086) moves relative to body 082 in a longitudinal direction of body 082, and the resulting state is maintained is as follows. That is, in the state shown in Fig. 7, shank 076 is pulled in an axial direction of insertion aid (bougie) 003, and engaging portion 078 is pulled out from notch 079. While that state is maintained, shank 076 (086) is rotated around a central axis in the axial direction of insertion aid (bougie) 003, and an edge of the tip side engaging portion 078 (088) and a side end portion of base end 070 (080) of body 072 (082) are abutted to each other. This compresses coiled spring 071 (081) in a length of a portion where notch 079 (089) and engaging portion 078 (088) are engaged, as shown in Fig. 8, whereby a state in which coiled spring 081 having the predetermined diameter can protrude in relation to the predetermined diameter on the base end side can be maintained. When the opposite operation is performed in the state shown in Fig. 8, it is also possible to release the pull, and easily return it to the state shown in Fig. 7. As described above, by operating control unit 085 (075) (in particular, shank 086 (076)), area (071 (081)) having the predetermined diameter can be deformed, whereby it protrudes in relation to the predetermined diameter (that is, area (071 (081)) having the predetermined diameter expands in a vertical direction to the axial direction of insertion aid (bougie) 003), or on the contrary, the protrusion can be released, that is, the area can return from the state in which the area is expands in the vertical direction to the axial direction of insertion aid (bougie) 003 to the original size.

In this embodiment, as shown in Fig. 7 and Fig. 8, the structure in which the control unit (including its forming members) is moved toward the base end side in the longitudinal direction of the insertion aid, and rotated, whereby the pull is maintained, or it is rotated as above and is moved toward the tip side in the longitudinal direction, whereby the pull is released, is adopted, but other structures such as the structure described in the first embodiment may be adopted.

In the third embodiment, as shown in Fig. 8, coiled spring 081 having the predetermined diameter is axially compressed by narrowing the space between body 082 and tip end area 084, and each element of the winding wire of coiled spring 081 shifts to linear member 083, whereby the coiled spring protrudes in relation to the predetermined diameter (that is, area (071 (081)) having the predetermined diameter expands in a vertical direction to the axial direction of insertion aid (bougie) 003). Properties of coiled spring (071, 081) such as a material, a coil diameter, a wire diameter, wire turns, and a spring constant should be appropriately decided depending on the insertion aid used and properties of wall surfaces of the inside of the medical device containing the same. If the spring constant is insufficiently large, however, the holding force cannot be obtained. It is, thus, at least 1 gf/mm (9.8 × 10⁻³ N/mm), preferably 5 gf/mm (49 × 10⁻³ N/mm) or more. If there is an insufficient clearance between the linear member and the coil inner diameter, the elements of the winding wire do not shift when the spring is compressed and thus the protrusion insufficiently occurs. For this reason, the difference between the outer diameter of linear member 083 and the inner diameter of coiled spring 081 is preferably eaqual to or more than an outer diameter of coil forming coiled spring 081. In order to sufficiently protrude the spring, the compression amount on the operation is preferably 1/3 the length of coiled spring 081 placed or more.

Coiled spring 071 (081), body 072 (082), linear member 073 (083), tip end area 074 (084), and control unit 075 (085), which are the members forming insertion aid (bougie) 033 in this embodiment, can be appropriately produced using known materials such as metals and various resins.

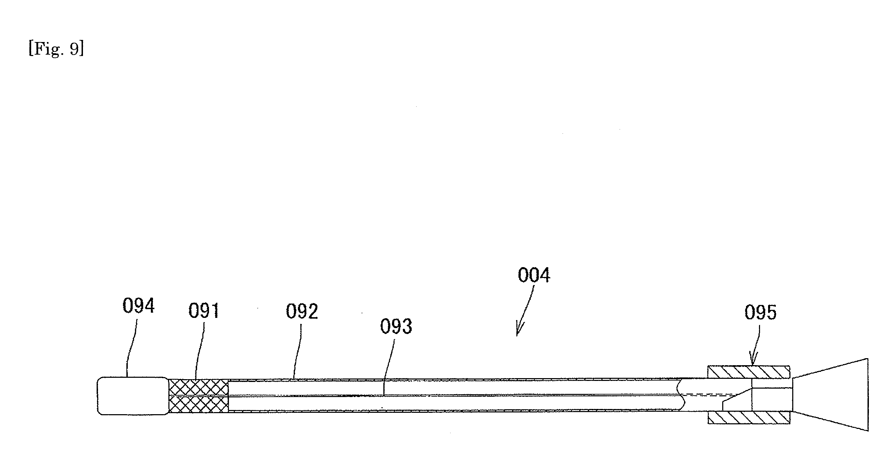

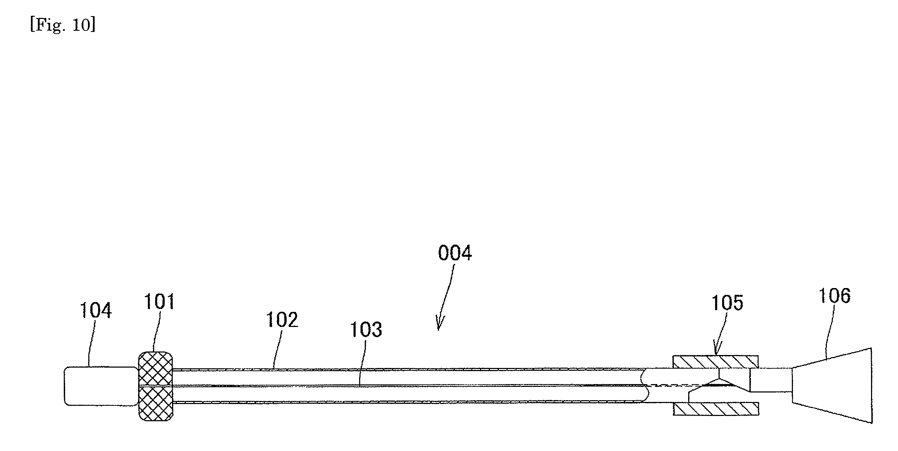

Figs. 9 and 10 show a fourth embodiment of an insertion aid according to the present invention.
Fig. 9 is a whole schematic view showing insertion aid (bougie) 004 according to the present invention used in a medical device treating a lacrimal duct. Rubber elastic tubular member 091 having a predetermined diameter is placed between body 092 of insertion aid (bougie) 004 used in a medical device treating a lacrimal duct and tip end area 094 of the body, through which linear member 093 which is connected to tip end area 094 and control unit 095 placed on the base end and passes through the inside of body 092, passes. Rubber elastic tubular member 091 having the predetermined diameter is placed between body 092 and tip end area 094 so that it has the predetermined diameter, which is the same as their outer diameters (sizes).
Control unit 095 substantially has the same structure as in the third embodiment, and thus detailed explanations are omitted.

Fig. 10 is a whole schematic view showing insertion aid (bougie) 004 according to the present invention used in a medical device treating a lacrimal duct. A state in which tubular member 101 having the predetermined diameter is compressed in the axial direction of insertion aid (bougie) 004, whereby it protrudes in relation to the predetermined diameter, is shown.

In Fig. 10, control unit 105 (in particular, shank 106) moves relative to body 102 toward the base end, while it pulls linear member 103 and tip end area 104 connected thereto. In this embodiment, the structure in which control unit 105 (shank 106) moves relative to body 102 is substantially the same as in the third embodiment, and thus the detailed explanations are omitted. Similar to the case of the third embodiment, the other embodiments explained in the first embodiment may by adopted as the structure of control unit (095, 105), in addition to the embodiment shown in Figs. 9 and 10.

In the fourth embodiment, as shown in Fig. 10, tubular member 101 having the predetermined diameter is axially compressed by narrowing the space between body 102 and flexible tip area 104, tubular member 101 deforms so that it expands in the diameter direction, whereby the tubular member protrudes in relation to the predetermined diameter (that is, area (091 (101)) having the predetermined diameter expands in a vertical direction to the axial direction of insertion aid (bougie) 004). Properties of tubular member (091, 101) such as a material, inner and outer diameters, and a length should be appropriately decided depending on the insertion aid used and properties of the inside wall surface of the medical device containing the same. As it is desired to return the diameter to the original predetermined diameter again after the deformation, rubber elastic materials are preferable. If the elastic modulus of the rubber elastic material is not small to some extent, sufficient protrusion cannot occur with respect to the compression. On the contrary, it is too small, holding force cannot be obtained. In view of these points, the bending elastic modulus of the rubber elastic material is preferably 285 MPa or less and 5 MPa or more, and it is more preferably 170 MPa or less and 12 MPa or more. The surface softness of tubular member 101 may be appropriately selected based on the properties of the inside wall surface of the medical device to be held, and when the material has softness to some extent, friction easily occurs and the holding property may be sometimes improved. The Shore D hardness, therefore, is preferably 64 or less, more preferably 27 or less.

Body 092 (102), linear member 093 (103), tip end area 094 (104), and control unit 095 (105), which are the members forming insertion aid in the fourth embodiment, can be appropriately produced using known materials such as metals and various resins.

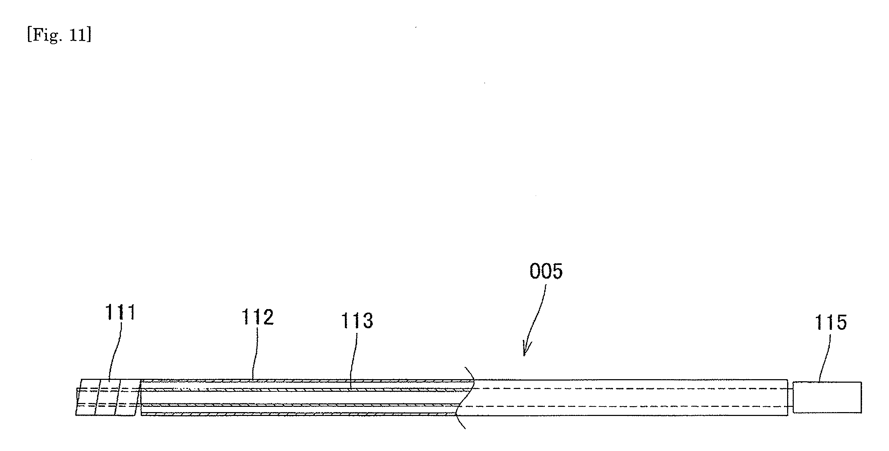

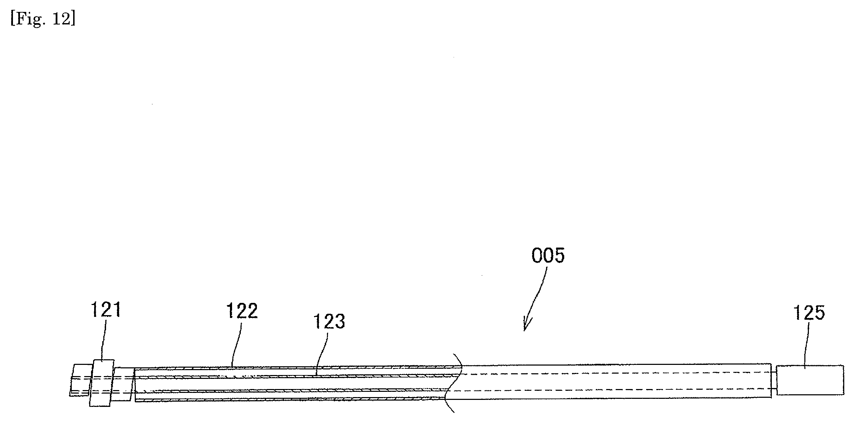

Figs. 11 and 12 show a fifth embodiment of an insertion aid according to the present invention.
Fig. 11 is a whole schematic view showing insertion aid (bougie) 005 according to the present invention used in a medical device treating a lacrimal duct. Body 112 of insertion aid (bougie) 005 used in the medical device treating a lacrimal duct is a tube made of, for example, stainless steel, and a part of its tip end is formed into tube 111 having the predetermined diameter, on which spiral notches are formed by spirally cutting it. Tube 111 spirally cut is placed in a state in which core material 113 passes through the inside thereof. Core material 113 is connected to control unit 115 placed at the base end and the most tip end of tube 111 placed at the tip area, and is placed in the state in which it passes through the inside of body 112. The most tip end of core material 113 is connected and fixed to the most tip end of tube 111. Tube 111 on which the spiral notches are formed by spiral cut can be integrally formed with the tip end area of body 112 by, for example, spirally cut the tip end of a stainless tube. Also, tube 111 may be formed by winding an elongated flat plate made of a metal or resin to form a spiral tube, and tube 111 obtained may be fixed to the tip end of body 112 in a known method.

Control unit 115 is connected to and fixed to the base end side of core material 113, and rotatably provided on the base end side of body 112 around core material 113 as the central axis. When tube 111 expands to the axial direction of body 112, (for example, a state shown in Fig. 12), in order to controll a size of the expanding tube and maintain the size, control unit 115 is formed so that a rotation angle is controlled and kept.

Fig. 12 is a whole schematic view showing insertion aid (bougie) 005 according to the present invention used in the medical device treating a lacrimal duct. A state in which tube 121 having the predetermined diameter, on which spiral notches are formed by spirally cutting, is unwound (that is, deforms) in a direction in which the spiral is loosened by control unit 125 and core material 123, whereby it protrudes in relation to the predetermined diameter (that is, area (111 (121)) having the predetermined diameter expands in a vertical direction to the axial direction of insertion aid (bougie) 005), is shown. In addition, it is possible to easily return the state in which control unit 125 is rotated to loosen the spiral and the tube expands in the vertical direction to the axial direction of insertion aid (bougie) 005 to the original state of the spiral as shown in Fig. 11. As described above, by operating control unit 115 or 125, area (111 (121)) having the predetermined diameter can be deformed, whereby it protrudes in relation to the predetermined diameter (that is, area (111 (121)) having the predetermined diameter expands in the virtical direction to the axial direction of insertion aid (bougie) 005), or on the contrary, the protrusion can be released, that is, the area can return from the state in which the area expands in the vertical direction of insertion aid (bougie) 005 to the original size.

Properties of tube 111 (121) on which the spiral notches are formed by spirally cutting such as a material, a shape of the spiral, and a pitch should be appropriately decided depending on the insertion aid used and properties of the inside wall surface of the medical device containing the same. In order to obtain a sufficient holding force, a material having a certain amount of strength, such as a stainless steal, is preferably used. If the pitch of the spiral is small, it may be difficult to obtain the holding strength, or a large number of rotations may be necessary for unwinding to increase the diameter, and therefore, the pitch is preferably 0.5 mm or more, more preferably 1 mm or more.

Body 112 (122), tube 111 (121), core material 113 (123), and control unit 115 (125), which are members forming the insertion aid in the fifth embodiment, can be appropriately produced using known materials such as metals and various resins other than the materials described above. In Figs. 11 and 12, as the core material, the tubular member is used, but a solid singly wire which is not tubular may be used as the material.

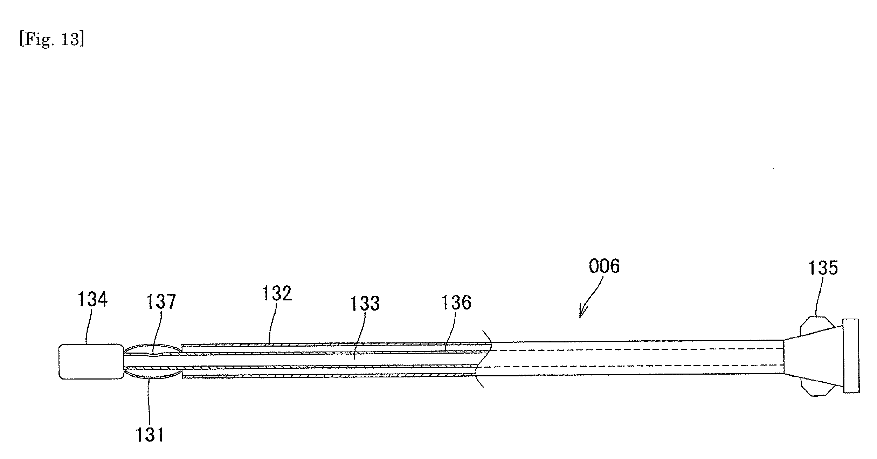

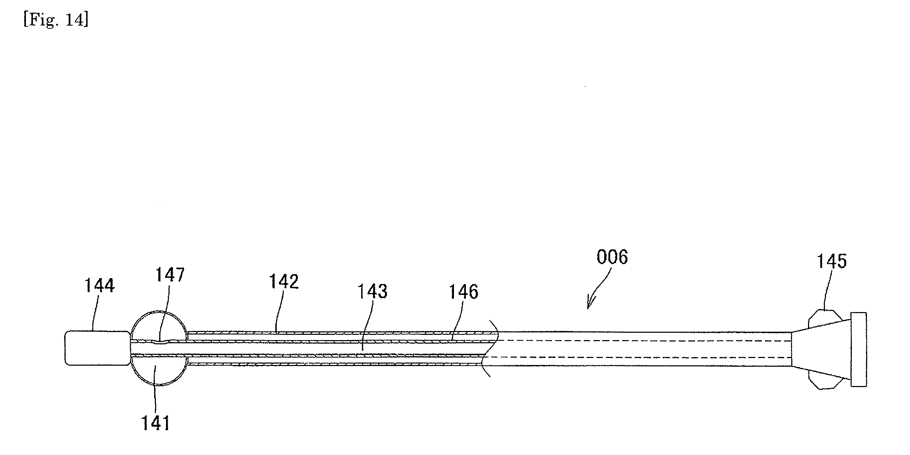

Figs. 13 and 14 show a sixth embodiment of an insertion aid according to the present invention.
Fig. 13 is a whole schematic view showing insertion aid (bougie) 006 according to the present invention used in a medical device treating a lacrimal duct. Balloon 131 which is expandable or shrinkable by a pressure is placed between body 132 of insertion aid (bougie) 006 used in a medical device treating a lacrimal duct and tip end area 134. In this embodiment, a structure in which a pressure can be introduced into balloon 131 through pressure lumen 133 communicating with expansion port 135 is employed. That is, inner tube 136 provided with pressure lumen 133 is placed inside body 132, and expansion port 135 having a lumen over its entire length on their base end sides. In addition, tip end area 134 is placed at the tip of inner tube 136 extending from the tip end of body 132, and balloon 131 is provided between a base end of tip end area 134 and a tip end of body 132 so that inner tube 136 is covered with the balloon. Further, small hole 137, which communicates pressure lumen 133 with the inside of balloon 131, is provided in the area in which inner tube 136 is covered with balloon 131. The inside of balloon 131 is communicated from an opening on the base end side of expansion port 135 through pressure lumen 133 and small hole 137 by the structure described above. A pressure is introduced into balloon 131 by injecting fluid, using, for example, a syringe from the base end side of expansion port 135, whereby the balloon can be expanded (that is, deforms). On the contrary, when the fluid is sucked from the inside of balloon 131, the introduced pressure can be lowered, whereby balloon 131 can be easily shrunk (that is, deforms). As described above, by operating the pressure control through expansion port 135, area (131) having the predetermined diameter can be deformed, whereby it protrudes in relation to the predetermined diameter (that is, area (131) having the predetermined diameter expands in the virtical direction to the axial direction of insertion aid (bougie) 006), or on the contrary, the protrusion can be released, that is, the area can return from a state in which the area expands in the vertical direction of insertion aid (bougie) 006 to the original size.

Fig. 14 is a whole schematic view showing insertion aid (bougie) 006 according to the present invention used in a medical device treating a lacrimal duct, and shows a state in which a pressure is introduced into balloon 141 which is expandable or shrinkable by a pressure and it protrudes in relation to the predetermined diameter (that is, area (131 (141)) having the predetermined diameter expands in the vertical direction to the axial direction of insertion aid (bougie) 006). Properties of balloon (131, 141) such as a material, a size, and a balloon compliance should be appropriately decided depending on the insertion aid used and properties of the inside wall surface of the medical device containing the same. In order to make a film thickness of the balloon as thin as possible, the material is preferably nylon or nylon elastomer. In order to maintain the pressure resistance, the compliance is preferably as small as possible, and it is preferably 1 mm/MPa or less. The compliance is defined as an increase (mm) of a diameter of a balloon per unit pressure (MPa). Expansion port 145 may have a valve or cock to maintain the expansion state of balloon 141, which is not shown in Fig. 14.

Body 132 (142), inner tube 136 (146), expansion port 135 (145), and tip end area 134 (144), which are the members forming the insertion aid in the sixth embodiment, can be appropriately produced using known materials such as metals and various resins.

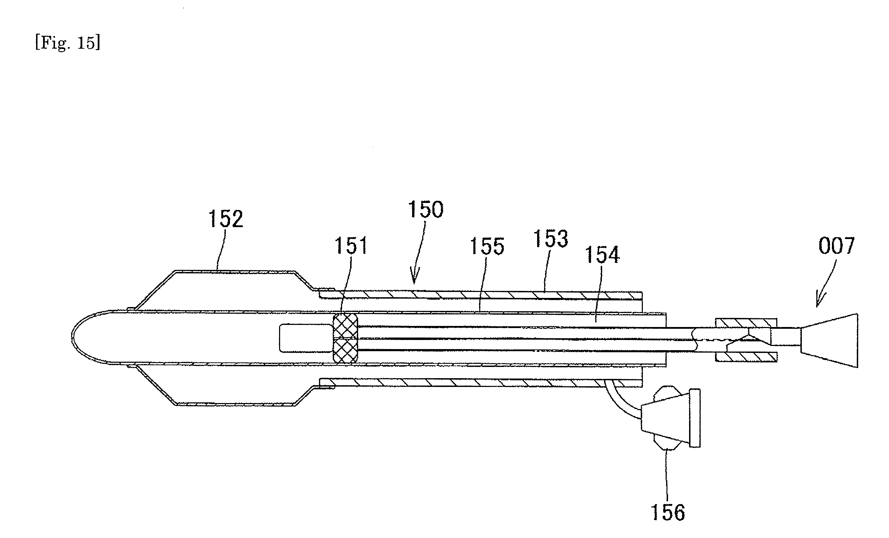

Fig. 15 is a schematic view showing a state in which insertion aid 007 according to the present invention used in a medical device treating a lacrimal duct is placed inside dilatation catheter 150 for treating a lacrimal duct, and holds dilatation catheter 150 for treating a lacrimal duct, which is the medical device treating a lacrimal duct, from the inside. In this view, the bougie of the fourth embodiment is used as the insertion aid.

Dilatation catheter 150 for treating a lacrimal duct is composed of dilatation part 152 for dilating a stenotic area, outer tube 153, inner tube 155 having lumen 154 for introducing the insertion aid, and expansion port 156 for introducing fluid to expand dilatation part 152. Fig. 15 shows a state in which tubular member 151 of insertion aid (bougie) 007 placed in the inside of dilatation catheter 150 for treating a lacrimal duct, that is, lumen 154 protrudes in relation to the predetermined diameter (that is, the area having a predetermined diameter expands in a vertical direction to the axial direction of insertion aid (bougie) 007) and presses an inside wall of inner tube 155, whereby insertion aid 007 holds dilatation catheter 150 for treating a lacrimal duct from the inside.

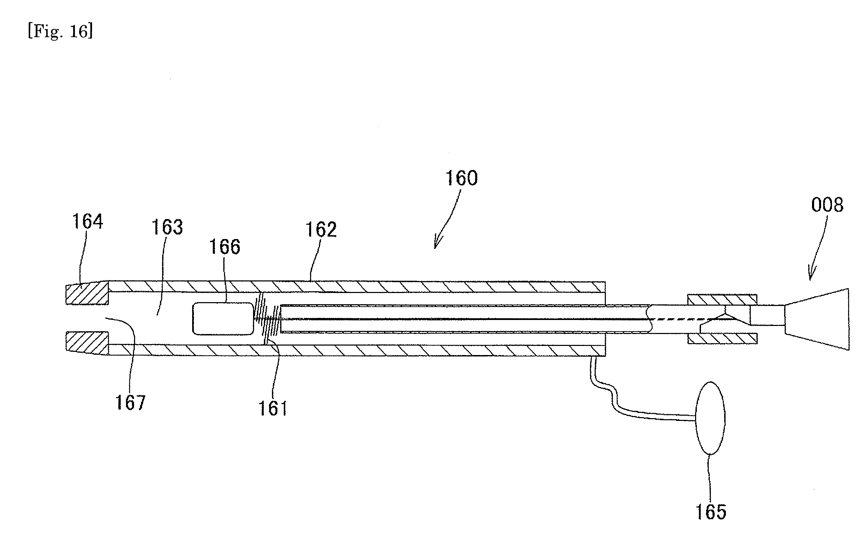

Fig. 16 is a schematic view showing a state in which insertion aid 008 according to the present invention used in a medical device treating a lacrimal duct is placed inside tube 160 for treating a lacrimal duct, and holds tube 160 for treating a lacrimal duct, which is the medical device treating a lacrimal duct, from the inside. In this view, the bougie of the third embodiment is used as the insertion aid.

Tube 160 for treating a lacrimal duct is composed of body portion 162 which is indwelled in a stenotic area, and stopper 165 in a punctum, and lumen 163 for passing insertion aid 008 is placed inside body portion 162. Tip end area 164 of body portion 162, which communicates with lumen 163 and opens at the most tip side, is provided at a tip of body portion 162. The size of opening 167 on the base end side of tip end area 164 is preferably smaller than the maximum width of the most tip of tip end area 166 of insertion aid (bougie) 008 so that tip end area 166 does not eject from the most tip end of the opening at tip end area 164 of body portion 162.

Fig. 16 shows a state in which coiled spring 161 of insertion aid (bougie) 008 which is plcaed inside tube 160 for treating a lacrimal duct, that is, in lumen 163, protrudes in relation to the predetermined diameter (that is, the area having the predetermined diameter expands in the vertical direction to the axial direction of insertion aid (bougie) 008) and press the surface of the inner wall forming lumen 163, whereby insertion aid (bougie) 008 holds tube 160 for treating a lacrimal duct from the inside.

A case in which tip end area 164 of body portion 162 opens is more likely to be preferable, because secretion products and contaminants are not indwelled therein, but it is necessary that the tip edge has an adequate strength, because insertion aid (bougie) 008 penetrates through the tip edge upon the insertion and the lacrimal duct is injured, or a structure in which it is difficult for the insertion aid to penetrate the tip edge is necessary, if the tip edge is low in the strength. Tube 160 for treating a lacrimal duct shown in Fig. 16 is excellent, because insertion aid (bougie) 008 presses tube 160 for treating a lacrimal duct from the inside and integrated with the tube, and not only the insertion operability is improved but also it is hard to shift the relative position. In addition, even if insertion aid (bougie) 008 is brought into contact with tip end area 164 of tube 160 for treating a lacrimal duct, there is a room in which tip end area 166 of insertion aid (bougie) 008 can move back, and there is a buffer effect (that is, coiled spring 161 deforms to absorb the pushing force), whereby the safety against the penetration is improved.

### Examples

Concrete Examples of the present invention will be explained in detail below.

### (Example 1)

A guide wire, which was an insertion aid used in a catheter for treating a stenotic vessel, having a structure shown in Fig. 1, was produced by combining a body of the guide wire of a stainless steel tubular member whose surface was coated with polytetrafluoroethylene, and having an outer diameter of 0.36 mm, an inner diameter of 0.22 mm, and a length of about 1600 mm; a flexible tip area of a stainless steel taper core wire around which a stainless steel coil member was wound, having an outer diameter of 0.36 mm and a length of about 200 mm; a stainless steel linear member having an outer diameter of 0.15 mm; a stainless steel coiled spring having a predetermined diameter of a coil diameter of 0.36 mm, and having a wire diameter of 0.07 mm, wire turns of 30, a spring constant of 5 gf/mm (49 × 10⁻³ N/mm), and a length of 6 mm; and a stainless steel control unit having an outer diameter 0.36mm.
In this guide wire, when the control unit was slid 3 mm toward the base end, as shown in Fig. 2, the coiled spring could protrude up to the maximum diameter of 0.55 mm in relation to the outer diameter of the guide wire of 0.36 mm (that is, the area having the outer diameter of 0.36 mm could deform and expand until the diameter reached at most 0.55 mm).
When this guide wire was used in a PTCA catheter of a 14 system (an applicable guide wire inner diameter: 0.014 inch (0.36 mm)), as shown in Fig. 3, it could hold the dilatation catheter, which was the catheter for treating a stenotic vessel, form the inside so that the relative position between the guide wire and the PTCA catheter was firmly fixed.

### (Example 2)

A guide wire, which was an insertion aid used in a catheter for treating a stenotic vessel, having a structure shown in Fig. 4, was produced by combining a body of the guide wire of a stainless steel cylindrical member whose surface was coated with polytetrafluoroethylene, and having an outer diameter of 0.36 mm, an inner diameter of 0.22 mm, and a length of about 1600 mm; a flexible tip area of a stainless steel taper core wire around which a stainless steel coil member was wound, having an outer diameter of 0.36 mm and a length of about 200 mm; a stainless steel linear member having an outer diameter of 0.15 mm; a tubular member having a predetermined diameter which was made of polyamide elastomer having a Shore hardness of D 27 and a bending elastic modulus of 12 MPa, and having an outer diameter of 0.36 mm, an inner diameter of 0.20 mm and a length of 4 mm; and a stainless steel control unit having an outer diameter 0.36mm.
According to this guide wire, when the control unit was slid 1 mm toward the base end, as shown in Fig. 5, the tubular member could protrude up to the maximum diameter of 0.47 mm in relation to the outer diameter of the guide wire of 0.36 mm (that is, the area having the outer diameter of 0.36 mm could deform and expand until the diameter reached at most 0.47 mm).
When this guide wire was used in a microcatheter for penetration of a 14 system (an applicable guide wire inner diameter: 0.014 inch (0.36 mm)), as shown in Fig. 6, it could hold the microcatheter, which was the catheter for treating a stenotic vessel, form the inside so that the relative position between the guide wire and the microcatheter was firmly fixed.

### (Example 3)

An insertion aid (bougie) used in a medical device for treating a lacrimal duct, having a structure shown in Fig. 7, was produced by combining a body of an insertion aid (bougie) of a stainless steel cylindrical member having an outer diameter of 0.48 mm, an inner diameter of 0.30 mm, and a length of 60 mm; a tip end area made of stainless steel and having an outer diameter of 0.48 mm, an inner diameter of 0.30 mm, and a length of 2 mm; a stainless steel linear member having an outer diameter of 0.18 mm; a stainless steel coiled spring having a predetermined diameter of a coil diameter of 0.48 mm, and having a wire diameter of 0.09 mm, wire turns of 40, a spring constant of 10 gf/mm (98 × 10⁻³ N/mm), and a length of 8 mm; and a control unit made of a styrene-butadiene material.
In this insertion aid (bougie), when the control unit was slid 3 mm toward the base end, as shown in Fig. 8, the coiled spring could protrude up to the maximum diameter of 0.70 mm in relation to the outer diameter of the body of the insertion aid (bougie) of 0.48 mm (that is, the area having the outer diameter of 0.48 mm could deform and expand until the diameter reached at most 0.70 mm).
When this insertion aid (bougie) was used in a polyurethane tube for treating a lacrimal duct having an inner diameter 0.52 mm, as shown in Fig. 16, it could hold the tube for treating a lacrimal duct from the inside. When the insertion aid (bougie) was inserted halfway into the lacrimal duct tube and used, the relative position to the lacrimal duct tube could be freely set and it was hard to shift it; that is, it had excellent operability. In addition, since the insertion aid (bougie) and the lacrimal duct tube were firmly fixed, the penetration through the tip was difficult upon the insertion, and a highly safe medical device for treating a lacrimal duct as shown in Fig. 16 could be obtained by the combination with the lacrimal duct tube having an open tip. Further, it was confirmed that the medical device is a medical device treating a lacrimal duct in which even if a very great force was applied to the tip end when the insertion aid is inserted into the lacrimal duct tube, the insertion aid (bougie) of the present aspect had a structure in which it was axially compressed through the coiled spring, and therefore the penetration was also difficult due to this buffer effect.

### (Example 4)

An insertion aid (bougie) used in a medical device for treating a lacrimal duct, having a structure shown in Fig. 9, was produced by combining a body of an insertion aid (bougie) of a stainless steel cylindrical member having an outer diameter of 0.48 mm, an inner diameter of 0.30 mm, and a length of 60 mm; a tip end area made of stainless steel and having an outer diameter of 0.48 mm, an inner diameter of 0.30 mm, and a length of 2 mm; a stainless steel linear member having an outer diameter of 0.18 mm; a tubular member having a predetermined diameter which was made of polyamide elastomer having a Shore hardness of D 27 and a bending elastic modulus of 12 MPa, and had an outer diameter of 0.48 mm, an inner diameter of 0.20 mm, and a length of 5 mm; and a control unit made of a styrene-butadiene material.
In this insertion aid (bougie), when the control unit was slid 1 mm toward the base end, as shown in Fig. 10, the tubular member could protrude up to the maximum diameter of 0.56 mm in relation to the outer diameter of the body of the insertion aid (bougie) of 0.48 mm (that is, the area having the outer diameter of 0.48 mm could deform and expand until the diameter reached at most 0.56 mm).
When this insertion aid (bougie) was used in a polyurethane tube for treating a lacrimal duct having an inner diameter 0.52 mm, as shown in Fig. 16, it could hold the tube for treating a lacrimal duct from the inside. When the insertion aid (bougie) was inserted halfway into the lacrimal duct tube and used, the relative position to the lacrimal duct tube could be freely set and it was hard to shift it; that is, it had excellent operability. In addition, since the insertion aid (bougie) and the lacrimal duct tube were firmly fixed, the penetration through the tip was difficult upon the insertion, and a highly safe medical device for treating a lacrimal duct as shown in Fig. 16 could be obtained by the combination with the lacrimal duct tube having an open tip. Further, it was confirmed that the medical device is a medical device treating a lacrimal duct in which even if a very great force was applied to the tip end, the insertion aid (bougie) of the present invention had a structure in which it is compressed in the axial direction through the tubular member, and therefore the penetration was also difficult due to this buffer effect.

### (Example 5)

An insertion aid (bougie) used in a medical device for treating a lacrimal duct, having a structure shown in Fig. 9, was produced by combining a body of an insertion aid (bougie) of a stainless steel cylindrical member having an outer diameter of 0.63 mm, an inner diameter of 0.50 mm, and a length of 170 mm; a tip end area made of stainless steel and having an outer diameter of 0.63 mm, an inner diameter of 0.50 mm, and a length of 5 mm; a stainless steel linear member having an outer diameter of 0.30 mm; a tubular member having a predetermined diameter which was made of polyamide elastomer having a Shore hardness of D 27 and a bending elastic modulus of 12 MPa, and had an outer diameter of 0.63 mm, an inner diameter of 0.30 mm and a length of 6 mm; and a control unit made of a styrene-butadiene material.
In this insertion aid (bougie), when the control unit was slid 1.5 mm toward the base end, as shown in Fig. 10, the tubular member could protrude up to the maximum diameter of 0.74 mm in relation to the outer diameter of the body of the insertion aid (bougie) of 0.63 mm (that is, the area having the outer diameter of 0.63 mm could deform and expand until the diameter reached at most 0.74 mm). When this insertion aid (bougie) was used in a dilatation catheter for treating a lacrimal duct, having a lumen with an inner diameter of 0.70 mm, as shown in Fig. 15, it could hold the dilatation catheter for treating a lacrimal duct from the inside so that the relative position between the insertion aid (bougie) and the dilatation catheter for treating a lacrimal duct could be firmly fixed.

### (Example 6)

A spiral tube having an outer diameter of 0.63 mm and a pitch of 0.6 mm at a part of a tip area of a body of an insertion aid (bougie), which was a stainless steel cylindrical member having an outer diameter 0.63 mm, an inner diameter 0.50 mm, and a length 165 mm was formed. Then, a stainless steel core material having an outer diameter of 0.36 mm, which was connected to a control unit placed at the base end and placed in the body inside is welded to the most tip of the spiral tube to produce an insertion aid (bougie) used in a medical device for treating a lacrimal duct, having a structure shown in Fig. 11.
In this insertion aid (bougie), as shown in Fig. 12, when the control unit was rotated, the spiral tube could protrude up to the maximum diameter of 0.88 mm in relation to the outer diameter of the body of the insertion aid (bougie) of 0.63 mm (that is, the area having the outer diameter of 0.63mm could deform and expand until the diameter reached at most 0.88mm).
When this insertion aid (bougie) was used in a dilatation catheter for treating a lacrimal duct having a lumen with an inner diameter of 0.70 mm as shown in Fig. 15, it could hold the dilatation catheter for treating a lacrimal duct from the inside so that the relative position between the insertion aid (bougie) and the dilatation catheter for treating a lacrimal duct could be firmly fixed.

### (Example 7)

An insertion aid (bougie) used in a medical device treating a lacrimal duct, having a structure shown in Fig. 13, was produced by combining a body of an insertion aid (bougie) of a stainless steel cylindrical member having an outer diameter of 0.63 mm, an inner diameter of 0.50 mm, and a length of 165 mm; a tip end area made of stainless steel and having an outer diameter of 0.63 mm, an inner diameter of 0.50 mm, and a length of 5 mm; a stainless steel tubular member having a pressure lumen with an outer diameter of 0.45 mm and an inner diameter of 0.25 mm; a dilatation part having a predetermined diameter which was made of polyamide elastomer having a Shore hardness of D 69, outer diameter of 0.63 mm, and a compliance of 0.2 mm[MPa; and an expansion port made of a styrene-butadiene material.
In this insertion aid, as shown in Fig. 14, when a pressure of 0.6 MPa was introduced into the dilatation part from the expansion port, the dilatation part could protrude up to 0.75 mm in relation to the outer diameter of the body of the insertion aid (bougie) of 0.63 mm, (that is, the area having the outer diameter of 0.63 mm could deform and expand until the diameter reached at most 0.75 mm).
When this insertion aid (bougie) was used in a dilatation catheter for treating a lacrimal duct having a lumen with an inner diameter of 0.70 mm as shown in Fig. 15, it could hold the dilatation catheter for treating a lacrimal duct from the inside so that the relative position between the insertion aid (bougie) and the dilatation catheter for treating a lacrimal duct could be firmly fixed.

### Reference Signs List

- 001: Guide wire of a first embodiment
- 002: Guide wire of a second embodiment
- 003: Insertion aid (bougie) of a third embodiment
- 004: Insertion aid (bougie) of a fourth embodiment
- 005: Insertion aid (bougie) of a fifth embodiment
- 006: Insertion aid (bougie) of a sixth embodiment
- 007: Insertion aid (bougie) of a fourth embodiment
- 008: Insertion aid (bougie) of a third embodiment
- 011: Coiled spring
- 012: Body of guide wire
- 013: Linear member
- 014: Flexible tip area
- 015: Control unit
- 021: Coiled spring
- 022: Body of guide wire
- 023: Linear member
- 024: Flexible tip area
- 025: Control unit
- 031: Coiled spring
- 036: Catheter for treating stenotic vessel (dilatation catheter)
- 037: Dilatation part
- 038: Outer tube
- 039: Inner tube
- 041: Tubular member
- 042: Body
- 043: Linear member
- 044: Flexible tip area
- 045: Control unit
- 051: Tubular member
- 052: Body
- 053: Linear member
- 054: Flexible tip area
- 055: Control unit
- 061: Fluid passage
- 066: Catheter for treating stenotic vessel (microcatheter)
- 067: Passage for passing guide wire
- 070: Side end portion of base end of body 072
- 071: Coiled spring
- 072: Body
- 073: Linear member
- 074: Tip end area
- 075: Control unit
- 076: Shank
- 077: Outer tube portion
- 078: Engaging portion
- 079: Notch
- 080: Side end portion of base end of body 082
- 081: Coiled spring
- 082: Body
- 083: Linear member
- 084: Tip end area
- 085: Control unit
- 086: Shank
- 087: Outer tube portion
- 088: Engaging portion
- 089: Notch
- 091: Tubular member
- 092: Body
- 093: Linear member
- 094: Tip end area
- 095: Control unit
- 101: Tubular member
- 102: Body
- 103: Linear member
- 104: Tip end area
- 105: Control unit
- 106: Shank
- 111: The most tip end of spirally cut tube
- 112: Body
- 113: Core material
- 115: Control unit
- 121: The most tip end of spirally cut tube
- 122: Body
- 123: Core material
- 125: Control unit
- 131: Balloon
- 132: Body
- 133: Pressure lumen
- 134: Tip end area
- 135: Expansion port
- 136: Inner tube
- 137: Small hole
- 141: Balloon
- 142: Body
- 143: Pressure lumen
- 144: Tip end area
- 145: Expansion port
- 146: Inner tube
- 147: Small hole
- 151: Tubular member
- 152: Dilatation part
- 153: Outer tube
- 154: Lumen for passing insertion aid
- 155: Inner tube
- 156: Expansion port
- 161: Coiled spring
- 162: Body portion
- 163: Lumen for passing insertion aid
- 164: Tip end area of body
- 165: Stopper
- 166: Tip end area
- 167: Opening of base end side of tip end area 164

## Claims

1. An insertion aid which is placed in a medical device and is used at the time when the medical device is inserted into a body, comprising a mechanism for holding the medical device from the inside.

2. The insertion aid according to claim 1, wherein the holding mechanism is a mechanism in which a part of the insertion aid having a predetermined diameter protrudes in relation to the predetermined diameter.

3. The insertion aid according to claim 2, wherein the holding mechanism is a mechanism in which a coiled spring having a predetermined diameter is placed on a part of the insertion aid, and the spring is axially compressed whereby it protrudes in relation to the predetermined diameter.

4. The insertion aid according to claim 2, wherein the holding mechanism is a mechanism in which a rubber elastic tubular member having a predetermined diameter is placed on a part of the insertion aid, and the tubular member is axially compressed whereby it protrudes in relation to the predetermined diameter.

5. The insertion aid according to claim 2, wherein the holding mechanism is a mechanism in which a spirally cut tube having a predetermined diameter is placed on a part of the insertion aid, and the spirally cut tube is radially unwound whereby it protrudes in relation to the predetermined diameter.

6. The insertion aid according to claim 2, wherein the holding mechanism is a mechanism in which a balloon expandable by pressure is placed on a part of the insertion aid, and a pressure is introduced into the balloon whereby it protrudes in relation to the predetermined diameter.

7. The insertion aid according to any one of claims 1 to 6, wherein the holding mechanism is a mechanism in which an area having a predetermined diameter placed on a part of the insertion aid is deformed by operation of a control unit placed on a base end whereby it protrudes in relation to the predetermined diameter.

8. A medical device comprising the insertion aid according to any one of claims 1 to 7.

9. The medical device according to claim 8, wherein the medical device is a catheter for treating a stenotic vessel.

10. The medical device according to claim 8, wherein the medical device is a dilatation catheter for treating a lacrimal duct.

11. The medical device according to claim 8, wherein the medical device is a tube for treating a lacrimal duct.
